# EUROPEAN PATENT APPLICATION

(11) **EP 0 747 705 A1**
(43) Date of publication of application: **11.12.1996**
(21) Application number: 96108409.2
(22) Date of filing: 28.05.1996
(51) Int. Cl.: G01N 33/574

(54) **Specific determination of NCA 90 glycoform in the blood of cancer patients**

(30) Priority: 09.06.1995 US 489188
(71) Applicant: Bayer Corporation, Pittsburgh, PA 15219-2502 (US)
(72) Inventor: Very, Donald L., Red Bank, New Jersey 07701 (US); Yeung, Kwok K., Ridgefield, Connecticut 06877 (US); Allard, William Jeffrey, Poughquag, New York 12570 (US)
(74) Representative: Kröger, Bernd, Dr.

(57) **Abstract**

It has been found that although both glycoforms of the cancer marker NCA 50/90 are expressed in normal tissue, only the 90 kDa glycoform is secreted into the bloodstream. An improved method is therefore provided for aiding in the diagnosis of, and monitoring the progression or course of cancer, particularly breast, colon, and lung cancer, and leukemia, in a patient by measuring the amount of NCA 90 specifically in a blood sample, e.g., serum sample, obtained from the patient.

## Description

### BACKGROUND OF THE INVENTION

The present invention relates to the diagnosis of cancer and to monitoring of the course of disease and therapy in cancer patients. More particularly, the invention relates to the specific determination of the 90 kDa glycoform of the cancer marker NCA 50/90 in blood.

The nonspecific crossreacting antigens (NCAs) are a group of sequence and structurally related proteins belonging to the carcinoembryonic antigen (CEA) gene family; a subfamily of the immunoglobulin supergene family (reviewed in 1-4). Encoded on the long arm of chromosome 19, the CEA gene family consists of approximately 29 genes (2-4), and is divided into two major subgroups: the CEA and the pregnancy specific β glycoprotein (PSβG) subgroups. The CEA subgroup contains 12 genes and pseudogenes (4, 5), and includes CEA (CD 66e), biliary glycoprotein (BGP, CD 66a), NCA 50/90 (CD 66c), NCA 95 (CGM6, CD 66b), CGM1 (CD 66d) (6), and CGM7 (7). To date, only the protein products of the CEA, BGP, NCA 50/90, and NCA 95 genes have been identified and biochemically characterized. The PSβG subgroup contains approximately 11 uniquely transcribed genes (4, 8, 9). A group of six additional genes belonging to a third subgroup have recently been described, but the degree of expression and tissue distribution of these gene products is unknown (5, 10). Reportedly, members of the CEA gene family function in granulocyte-endothelial cell adhesion, adhesion to bacterial fimbriae, and may play an accessory role in binding to Type I collagen (reviewed in 2, 4).

The product of the NCA 50/90 gene is a polypeptide of approximately 34 kDa, which is attached to the cell membrane via a glycosyl phosphatidylinositol linkage. Like other members of the CEA gene family, the peptide backbone of the NCA molecule is comprised of a series of disulfide-linked, immunoglobulin (Ig)-like domains. NCA 50/90 contains three such Ig-like domains: an N-terminal domain of 108-110 amino acids, a C region-like domain of 92-96 amino acids (referred to as an A domain), and a second C region-like domain of 86 amino acids (B domain). Post-translational glycosylation of the 34 kDa polypeptide results in a modified polypeptide with apparent molecular weights of 50 kDa (NCA 50; 50 kDa glycoform) and 90kDa (NCA 90; 90 kDa glycoform) (1-4).

Historically, the examination of NCA tissue expression has proceeded along two parallel lines of investigation: immunohistochemical, through the use of polyclonal and monoclonal antibodies; and molecular, through examination of the expression of NCA mRNA. Several laboratories have examined the expression NCA mRNA in normal and malignant tissue by Northern blot hybridizations. Employing this technique, NCA mRNA has been demonstrated in normal lung tissue (11, 12); in malignant lung (11), colon (13-15), breast (15); and in the proliferating cells associated with chronic myelogenous leukemia (15). Alternatively, immunohistochemical analysis has been used to demonstrate the expression of NCA in normal colon (13-16); in malignant breast (17), lung (19), colon (16); and in a pancreatic cell line (19).

The molecular cloning of the CEA gene family members into expression vectors has provided valuable tools for use in the analysis of anti-CEA and anti-NCA monoclonal antibody specificity and NCA 50/90 tissue distribution. Barnett et al. cloned and expressed the cDNAs encoding the NCA 50/90 and CEA genes (20, 21). These reagents were used to characterize the specificity of MAb 228, an antibody specific for both the 50 kDa and 90 kDa glycoforms of NCA 50/90, which does not cross-react with other CEA gene family members (22).

Recent studies have suggested that serum and plasma measurements of NCA 50/90 may have clinical utility in the management of patients with chronic myelogenous or lymphocytic leukemia (23); and cancer of the breast, colon, or lung (22). See also European Patent Publication Nos. 549,974; 621,480 and 628,820. These studies employed an ELISA which utilized a MAb (MAb 228) specific for both the 50 kDa and 90 kDa glycoforms of NCA 50/90. As currently formatted, this ELISA measures "total" NCA 50/90 in patient samples due to the specificity of MAb 228 for both NCA 50/90 glycoforms. As such, this assay does not determine which glycoform of NCA 50/90, if any, predominates in the circulation during malignancy.

Other laboratories have examined the normal and malignant tissue expression of NCA 50/90. Robbins et. al. (24) used mouse cell lines transfected with human CEA gene family members to determine the specificity of a panel of monoclonal antibodies. Using an NCA-specific MAb designated B6.2, Robbins demonstrated immunohistochemically that nearly all breast and lung tumors tested expressed NCA. Baranov et. al. (25) demonstrated the precise localization of NCA 50 in normal colonic mucosa and colonic adenocarcinoma. Using an indirect immunoperoxidase electron microscopy technique and a monoclonal antibody specific for the 50 kDa glycoform of NCA 50/90, Baranov et. al. detected the presence of NCA 50 on the apical surface and in microvesicles associated with the apical surface of normal human colon. Additionally, NCA 50 was detected in the spaces between adjacent microvilli, as internal components of goblet cells, and was found associated with the luminal surface of mature columnar epithelial cells. While these studies demonstrated detectable amounts of NCA 50/90 in normal lung and colon tissue, no results were offered regarding the relative distribution of the NCA 50/90 glycoforms in normal and malignant specimens.

### SUMMARY OF THE INVENTION

It has now been unexpectedly found that while both glycoforms are expressed in normal tissues, the 90 kDa glycoform of NCA 50/90 is predominately secreted into the bloodstream. The present invention therefore provides a method for measuring the human protein expressed by the NCA 50/90 gene which is secreted into the bloodstream of a patient, comprising measuring NCA 90 specifically in a blood sample obtained from the patient. Furthermore, since NCA 90 blood levels have been found to correlate with the blood levels of NCA 50/90 in both normal and cancer patients, the detection of NCA 90 specifically in blood will provide improved utility in the diagnosis and monitoring of cancers for which measurement of NCA 50/90 has been found to be of clinical value. The present invention thus provides the basis for a second generation diagnostic assay of greater clinical utility based on the specific detection of the predominant NCA glycoform in blood, e.g., serum or plasma.

More particularly, the present invention provides a method for aiding in the diagnosis of various cancers such as breast, colon, and lung cancer, and leukemia, in a patient who presents with symptoms of cancer (i.e., a symptomatic patient), comprising the steps of determining the amount of NCA 90 specifically in a blood sample obtained from the patient and comparing such measured amount to the mean amount of blood NCA 90 in the normal population, whereby the presence of a significantly increased higher amount of NCA 90 in the patient's blood is an indication of cancer in the patient. With this and other information suggestive of cancer, the physician is assisted in making a diagnosis.

The present invention also provides a method for monitoring the course or progression of various cancers, such as breast, colon, and lung cancer, and leukemia, in a patient who has been diagnosed with cancer. A series of specific immunoassays are performed over time to determine changes in the level of NCA 90 specifically in blood samples obtained from such patient whereby changes in the NCA blood level correlate with changes in disease status. More particularly, increases in blood NCA 90 levels will generally indicate a deteriorating condition while decreases in blood NCA 90 levels indicate an improving condition. Where the diagnosed patient has been treated for cancer, e.g., radiation, chemotherapy, surgery, or the like, to eliminate clinically detectable disease, increases in blood NCA 90 levels indicate possible recurrence of disease.

### BRIEF DESCRIPTION OF THE DRAWINGS

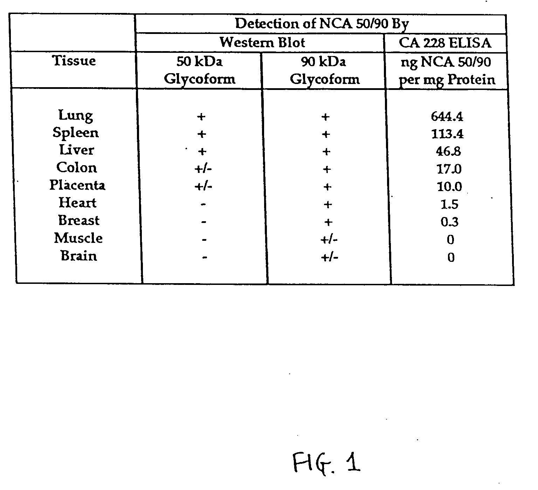

***Figure 1*. Detection of NCA 50/90 Gene Products in Normal Tissues by ELISA and Immunoprecipitation.** The presence of NCA 50/90 gene products in normal human lung, spleen, liver, colon, placenta, heart, breast, muscle, and brain tissue was examined using an ELISA assay (CA 228 ELISA) to measure total NCA 50/90 protein, and an immunoprecipitation-Western blotting technique to detect specific NCA 50/90 glycoforms. These procedures are described in detail in Examples: Materials and Methods. Both of these procedures utilized MAb 228, a monoclonal antibody specific for both the 50 kDa and 90 kDa glycoforms of the NCA 50/90 gene and non-crossreactive with other CEA gene family members. The amount of NCA 50/90 protein detected in the various tissues by ELISA is reported as ng NCA 50/90 per mg of tissue protein. The detection of specific NCA 50/90 glycoforms by immunoprecipitation and Western blotting is indicated using a qualitative scale as follows: "-", no detection of NCA glycoform; "+ /-", trace amount of NCA glycoform detected; and "+" , significant amount of NCA glycoform detected.

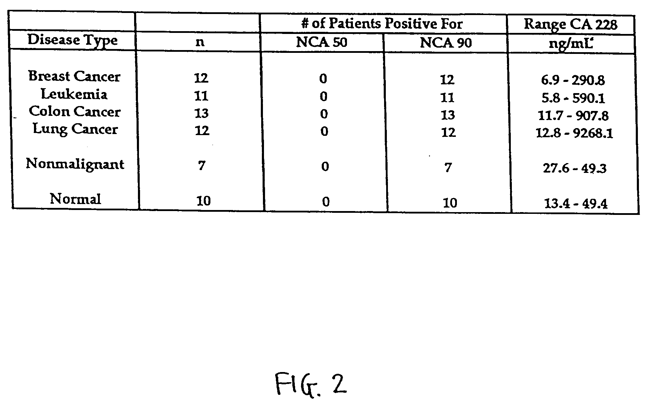

***Figure 2*****. Detection of NCA 50/90 Gene Products in the Sera of Normal, Nonmalignant Disease, and Cancer Patients by ELISA and Immunoprecipitation.** The presence of NCA 50/90 gene products in the sera of normal, nonmalignant disease, and cancer patients was determined by CA 228 ELISA and immunoprecipitation-Western blotting as described in Materials and Methods. The number of patients of each disease type is indicated. For each disease type, CA 228 ELISA results are reported as a range of lowest patient value to highest patient value. The number of patient serum samples which were positive for a specific NCA 50/90 glycoform by immunoprecipitation-Western blotting is also indicated.

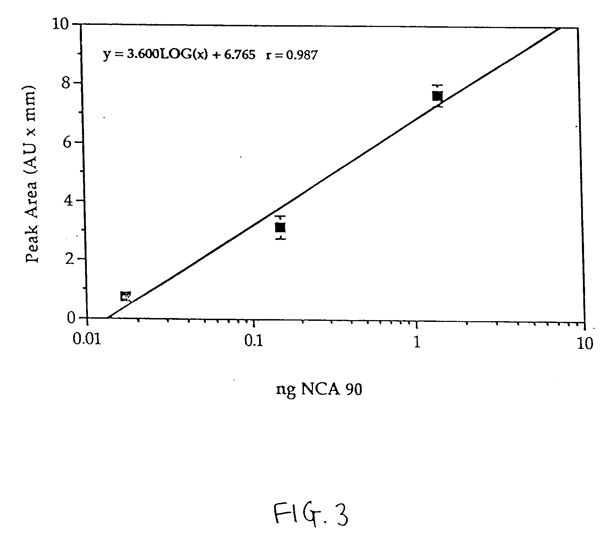

***Figure 3*****. Standard Curve Used in the Quantitation of NCA 90 Immunoprecipitated from Patient Samples.** Figure 3 presents a typical standard curve used in the quantitation NCA 90 serum immunoprecipitates. The amount of NCA 90 in three serial dilutions of lung tissue homogenate was determined by electrophoresis, immunoelectroblotting, and densitometric scanning as described below. Results are plotted as the mean ± 1 SD of four independent determinations. The range of the standard curve is from 0.017 ng to 1.44 ng.

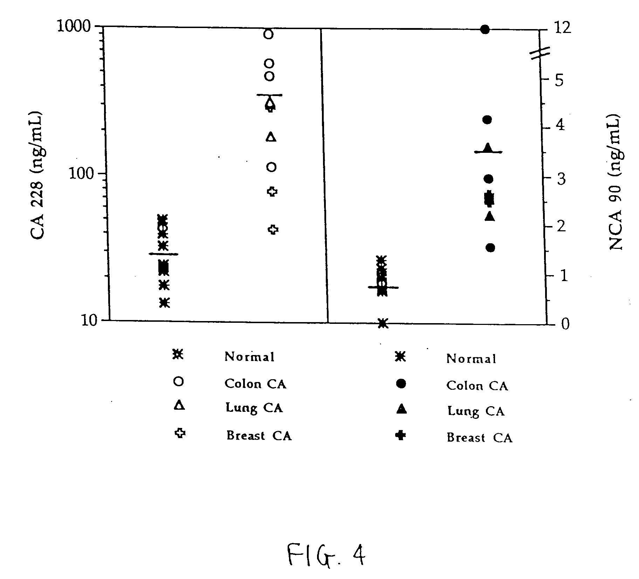

***Figure 4*****. Comparison of Serum CA 228 (Total NCA 50/90) and NCA 90 Values in Normal Subjects and Cancer Patients.** CA 228 and NCA 90 levels in the sera of a panel of 10 normal subjects and 9 cancer patients were determined by ELISA and immunoelectroblotting as described below. All cancer patients were diagnosed by attending physicians as possessing clinically active disease.

### DESCRIPTION OF THE PREFERRED EMBODIMENTS

In making the present invention, a study was performed to determine which glycoforms of NCA 50/90 are present in normal tissue, normal sera, cancer patient sera, and non-malignant disease patient sera. As reported in more detail in the Examples which follow, we have demonstrated that although both NCA 50/90 glycoforms are expressed in normal tissues, only the 90 kDa glycoform is detected in the circulation. The CA 228 ELISA, performed on acetone-extracted, normal, human tissue preparations, identified large amounts of NCA 50/90 (per mg tissue) in lung, and lesser amounts in spleen, liver, colon, placenta, heart, and breast tissue. The expression of specific NCA 50/90 glycoforms in these tissue preparations was determined by immunoprecipitation with an anti-NCA 50/90-specific MAb (MAb 228) conjugated to Affigel 10 and detection, following electroblotting, with a polyclonal rabbit anti-CEA antisera. Normal lung, spleen, liver, colon, and placental extracts expressed both NCA 50/90 glycoforms. MAb 228-Affigel immunoprecipitation of the serum of 48 patients with leukemia or cancer of the breast, lung, or colon detected the presence of only the 90 kDa glycoform in all samples. Additionally, only NCA 90 was detected in the serum of seven infectious or non-malignant disease patients, and ten normal subjects by immunoprecipitation. In all patient samples immunoprecipitated, the amount of the 90 kDa glycoform identified by immunoprecipitation, positively correlated with the serum level of total NCA 50/90 determined by ELISA.

The finding that only the 90 kDa glycoform of NCA 50/90 is found in the circulation provides the basis for NCA cancer marker assays of greater sensitivity and specificity in the detection and monitoring of malignancy by the specific measurement of the NCA 90 glycoform in blood.

Essentially any method may be employed in the measurement of blood (e.g., serum or plasma) NCA 90 levels. Typically, such measurement will be performed by sandwich immunoassay using two antibody reagents, one of which recognizes NCA 90 to the exclusion of NCA 50 and other related members of the CEA family (e.g., NCA 95, CEA, BGP, PSG, and NCA 2), while the other is capable of binding specifically or nonspecifically with NCA 90. Assay format and methods for the preparation of the required antibody reagents can be selected by the skilled worker in the field. Suitable antibody reagents can be labeled, e.g., enzyme-labeled, or immobilized, e.g., coated onto a microtiter plate, bound to plastic or magnetic beads or particles, and can be comprised of whole immunoglobulins, e.g., IgG or IgM, or fragments, e.g., Fab, Fab', and F(ab')₂ fragments, or aggregates thereof.

Preferably, the NCA 90 specific antibody reagent is prepared by immunization of a host animal with a suitable immunogen such as an NCA 90-containing immunogen mixture, e.g., a purified extract of spleen or tumor cells; NCA 90-expressing transfectant cell lines (see European Patent Publication 346,702); an immunogen conjugate comprising a synthetically prepared peptide coupled to a conventional immunogenic carrier molecule, where the peptide has an amino acid sequence encompassing a unique epitope of NCA 90; and the like as will be understood in the art. Antibody reagents comprising monoclonal antibodies will be generally preferred.

It will be understood that, similar to other types of accepted disease diagnostic and monitoring methods, the present method will not be useful on every patient diagnosed with cancer. Rather, the physician will use NCA 90 blood values in combination with other diagnostic values and clinical observations to diagnose the onset of cancer, and further to develop a course of treatment and therapy for each individual patient. It is also contemplated that monitoring blood levels of NCA 90 will provide a means for monitoring the progress of a course of therapy for an individual patient.

The present invention will now be illustrated, but is not intended to be limited by, the following examples.

### EXAMPLES

### MATERIALS AND METHODS

***Antibodies*** ***and Purified Proteins.*** Monoclonal antibody 228.2 (IgG₁, k) (ATCC HB11204) is specific for NCA 50/90. It does not crossreact with CEA, BGP, NCA 95, or PSβG. The production, purification, and specificity of this antibody has been described previously (22). Chromatographically purified mouse IgG used for conjugation to Affigel was purchased from Zymed Laboratories Inc. (San Francisco, California, USA) (catalog # 02-6502). Rabbit IgG (catalog # I-5006) used as a control first antibody for Western blots, goat anti-rabbit IgG conjugated to alkaline phosphatase (catalog # A-7539) used to detect immune complexes on Western blots, and a goat anti-mouse IgG-alkaline phosphatase conjugate (catalog # A-1418) used to detect immune complexes on commercially prepared Western blots were purchased from Sigma Chemical Co., St. Louis, Missouri, USA. Purified rabbit anti-human CEA used as the first antibody in Western blots was purchased from Dako (Carpinteria, California, USA) (Code # A-115).

***Patient*** ***Samples.*** Normal serum samples used in this study were obtained from healthy volunteers at the Diagnostics Business Group of Bayer Corporation, Tarrytown, New York, USA, and from the Hudson Valley Blood Services of Valhalla, New York, USA. Dr. Herbert Fritsche of the M.D. Anderson Cancer Center in Houston, Texas, USA, and Dianon Systems of Stamford, Connecticut, USA, supplied the serum samples from patients with breast, lung, or colon cancer. Serum from patients with leukemia were obtained from Oncology/Hematology Consultants (Memphis, Tennessee, USA).

All cancer patient samples used in this study were obtained from patients with active disease as determined by the attending physician based on physical examination and medical records.

***Preparation of Tissue Extracts***. Acetone extracted lyophilized human tissues were obtained from Calzyme Chemical Co. (San Luis Obispo, California, USA). Human heart tissue was purchased from American Biochemicals Inc. (San Diego, California, USA). One hundred milligrams of acetone powder was dissolved in 5 ml of cold homogenization buffer consisting of 50 mM HEPES (pH 7.4), 0.2 % NP-40, 0.5 mM pefablock (Boehringer Mannheim, Indianapolis, Indiana, USA) (catalog # 1429-868), 1 µM leupeptin (Boebringer Mannheim, catalog # 1017-101), 1 µM pepstatin (Boebringer Mannheim, catalog # 600-160), and 1 mM EDTA. Powder was then polytron homogenized on ice until the solution was homogenous (approximately 2-4 minutes). Following an additional 5 minute incubation, the homogenate was centrifuged at 5,000 rpm and 4°C for 5 minutes. The supernatant fluid was collected, aliquoted, and stored at -80°C. Total protein in the tissue homogenates was determined using the Pierce BCA Protein Assay according to the manufacturer's instructions (Pierce, Rockford, Illinois, USA).

***NCA 50/90 ELISA*****.** The NCA 50/90 ELISA (referred to as CA 228) was performed as previously described (23). Briefly, 96 well Immulon 4 plates (Dynatech, Chantilly, Viginia, USA) were coated with 50 µl per well of MAb 228.2 at 5 µg/ml in 0.1 M NaHCO₃, pH 9.0 and incubated overnight at 4°C. Unreacted sites in the wells were then blocked by the addition of 200 µl per well of buffer containing 25 mM Tris (pH 7.5), 150 mM NaCl, 0.05 % Tween-20, 0.1 % NaN₃ (TBST), and 5 % bovine serum albumin (BSA, fraction V, Sigma, Catalog # A-7030). Plates were blocked for 1 hour at 37°C. Plates were washed 6 times with TBST and 50 µl of patient sample of NCA 50/90 calibrator solution diluted 1:51 in 25 mM HEPES (pH 7.0), 250 mM NaCl, 100 µg/ml mouse IgG, 2.5 % BSA, 50 µg/ml gentamycin, and 0.1 % NaN₃ (sample diluent) was added to each well. Plates were then incubated for 2 hours at 37°C. Following washing, 50 µl of a solution of goat anti-CEA-alkaline phosphatase conjugate diluted to 2.5 µg/ml in 50 mM HEPES (pH 7.0), 150 mM NaCl, 1 mM MgCl₆H₂O, 0.1 mM ZnCl₂, 1 % BSA, 50 µg/ml gentamycin, and 0.1 % NaN₃ (conjugate diluent) was added to each well. After 1 hour incubation at 37°C and washing in TBST, 50 µl of a 1 mg/ml solution of p-nitrophenyl phosphate (Pierce, Catalog # 34074) in 1X diethanolamine substrate buffer (Pierce, Catalog # 34064) was added to each well and incubated at 37°C for 30 minutes. The reaction was terminated by the addition of 100 µl of 1 N NaOH to each well. Absorbance at 405 nm minus the absorbance at 490 nm was determined using a Thermoₘₐₓ microplate reader (Molecular Devices Corp., Sunnyvale, California, USA). The amount of NCA 50/90 in each sample was determined by comparison with a calibration standard curve using a nonlinear spline curve-fitting program.

***Conjugation of Mouse IgG and MAb 228 to Affigel 10*****.** Purified mouse IgG (Zymed Laboratories Inc.; catalog #02-6502) and MAb 228 were diluted in 0.1 M HEPES, pH 8.0 to a concentration of 1.5 mg/ml. A small aliquot of each solution was removed for protein determination. Approximately 2 ml of Affigel 10 (BioRad Laboratories, Richmond, California, USA; catalog #153-6046) was placed in each of two polypropylene 12 x 75 capped culture tubes, and was washed three times in cold deionized water by centrifugation at 5000 rpm for 3 minutes at 4°C. After the first centrifugation, Affigel solution was added or removed from the tube in order to obtain approximately 1.0 ml of packed gel in each tube. Following the last wash, the supernatant was removed from the Affigel pellet. Conjugation was performed by the addition of 2 ml of a 1.5 mg/ml antibody solution (3.0 mg) to 1.0 ml of packed Affigel. The Affigel was resuspended in the antibody solution by gentle agitation and was rocked for 4 hours at 4°C. Following conjugation incubation, each tube was centrifuged at 2500 rpm for 5 minutes at 4°C to pellet the IgG-Affigel conjugate. A small aliquot of the supernatant was removed for protein determination. The IgG-Affigel conjugates were resuspended and stored at 4°C. The amount of antibody conjugated to the Affigel was determined by BCA Protein Assay (Pierce) quantitation of the amount of protein in the preconjugation aliquot minus the amount in the postconjugation antibody solution. Typically, this conjugation protocol resulted in 1.25-1.5 mg Ab per ml of gel.

***Immunoprecipitation*****.** Prior to immunoprecipitation, tissue homogenates were diluted to 1.7 mg/ml in a sample diluent solution containing 100 mM NaH₂PO₄ (pH 7.4), 500 mM NaCl, and 2% Chaps detergent (Sigma Chemical Co.) Muscle (1.2 mg/ml) and brain (0.8 mg/ml) tissue preparations were used undiluted. IgG-Affigel beads were washed three times in PBS by centrifugation at 2500 rpm for 3-5 minutes. Pelleted gel was resuspended with and equal volume of PBS to a final concentration of 50%. Each sample and tissue homogenate was immunoprecipitated with both the MAb 228-Affigel conjugate and the control mouse IgG-Affigel conjugate. Immunoprecipitations were performed by the addition of 50 µl of undiluted patient serum or diluted tissue homogenate, 50 µl of sample diluent, and 30 µl of the 50% IgG-Affigel slurry to a 1.5 ml Eppendorf centrifuge tube. Following incubation at 4°C for 4 hours with gentle rocking, precipitates were washed three times in 0.5 ml of washing buffer containing 100 mM NaH₂PO₄ (pH 7.4), 0.1% SDS, 300 mM NaCl, and 2% Chaps. After the last wash, immunoprecipitates were resuspended in 25 µl of 2X SDS-PAGE sample buffer (see below) which did not contain dithiothreitol (DTT). Samples were stored at -20°C.

***Electrophoresis and Electroblotting*****.** Electrophoresis and Western blotting were performed essentially as previously described (26, 27). Purified proteins or immunoprecipitates were dissolved in an equal volume of 2X SDS-PAGE sample buffer containing 190 mM Tris (pH 6.8), 4 % SDS, 2 mM EDTA, 20 % glycerol, and 0.004 % bromophenol blue, and boiled for 5 minutes in a water bath. Electrophoresis was performed on a 4-20 % gradient gel (Daiichi-Enprotech, Natick, Massachusetts, USA) for 3-4 hours at 25-40 mA per gel. Proteins were electroblotted onto 0.2 micron PVDF membranes (BioRad) using a semi-dry electroblotting apparatus (Integrated Separations Systems-Enprotech, Natick, Massachusetts, USA) and ISS protein electroblotting buffers (Integrated Separation Systems, catalog # SS110204). Electroblotting was performed for 45 minutes at a constant voltage of 1 mA per cm² of membrane. Blotted membranes were then sequentially treated with PBS containing 3 % BSA, rabbit IgG or rabbit anti-human CEA at 1 µg/ml (first antibody), and goat anti-rabbit IgG-alkaline phosphatase at a 1:5000 dilution. Bound immune complexes were visualized by the addition of 5-bromo-4-chloro-3-indoyl phosphate and nitro blue tetrazolium (BCIP-NBT, Sigma, catalog # B-5655) according to the manufacturer's instructions.

Band-staining intensity (peak area) on the membranes was determined using a Pharmacia LKB UltroScan XL scanning densitometer.

***Quantitation of NCA 90*****.** Th amount of NCA 90 present in 50 µl of patient serum was determined by comparison of a sample's NCA 90 peak area to a standard curve of serial dilution of lung tissue homogenate of known NCA 90 concentration. Briefly, serial dilutions of non-immunoprecipitated lung tissue homogenate (of known NCA 50/90 concentration as determined by CA 228 ELISA) were electrophoresced and immunoblotted on PVDF membranes as described. NCA 90 concentration in each lung tissue dilution was determined by multiplication of the total NCA 50/90 concentration of the tissue dilution by the percentage of total NCA 50/90 which was NCA 90 (obtained from the densitometric scanning of the blot). A standard curve was then constructed using the log of NCA 90 concentration plotted versus the NCA 90 peak area (a representative standard curve is presented in Fig. 3). The amount of NCA 90 in 50 µl samples of patient sera was then quantitated by comparison of the NCA 90 peak area with the standard curve. The results were multiplied by 20 to determine the concentration of NCA 90 per ml of serum, and results are reported as ng NCA 90/ml.

### RESULTS

***Expression*** ***of NCA 50/90 in Normal Tissues.*** Despite extensive biochemical characterization, little is known regarding the normal tissue expression of NCA 50/90, primarily due to a lack of NCA 50/90-specific reagents. To examine the expression of NCA 50/90 in normal tissues, we used both an ELISA assay (CA 228 ELISA) and an immunoprecipitation protocol. Both methods utilize a monoclonal antibody, MAb 228, which is specific for both the glycosylated NCA 50 and NCA 90 gene products, and does not cross-react with other CEA gene family members (22). Using the CA 228 ELISA, we detected NCA 50/90 in the acetone powder extracts of lung, spleen, liver, colon, placenta, heart, and in a homogenate of breast tissue. No NCA 50/90 was detected in the muscle and brain tissue extracts (Figure 1). Curiously, NCA 50/90 was not found in muscle tissue, but was detected in heart tissue extracts. Additionally, the spleen was found to contain a greater amount of NCA 50/90 per mg protein than most of the other organs. A possible explanation for both of these findings is that the NCA 50/90 detected in heart and spleen tissue is derived from blood-borne granulocytes, known to express NCA 50/90 (23, 28-30), which was present in the organs at the time of tissue processing. The ELISA results shown in the table in Figure 1 demonstrate that lung, spleen, and liver tissue contain the greatest amount of total NCA 50/90 per mg of tissue protein.

To detect the specific glycoforms of NCA 50/90 expressed in normal tissues, acetone powder extracts and a preparation of breast tissue homogenate were immunoprecipitated with MAb 228-Affigel or mouse IgG-Affigel (control) prior to electrophoresis, and immunoblotting with either a rabbit anti-CEA or rabbit IgG antibody preparation (control).

The results of NCA 50/90 immunoprecipitation from tissue extracts or homogenates are summarized in the table in Figure 1. Lung, spleen, liver, colon and placenta, expressed both glycoforms of NCA 50/90. Heart and breast tissue expressed only NCA 90, while muscle and brain possessed barely detectable amounts of NCA 90. The failure to detect NCA 50 in heart, breast, muscle, and brain tissue implies either a lack of this glycoform in these tissues, or suggests that the sensitivity limit of NCA 50 detection by immunoprecipitation is approximately 10 ng of total NCA 50/90 per mg of tissue protein.
***Expression of NCA 50/90 In Cancer Patients Serum*****.** MAb 228 immunoprecipitation studies demonstrated the expression of both NCA 50 and 90 glycoforms in normal lung, spleen, liver, colon and placental tissue extracts, but only NCA 90 was found in heart, breast, muscle, and brain tissue. To determine the glycoforms of NCA 50/90 found in the serum of cancer patients, serum from 12 lung cancer, 13 colon cancer, 12 breast cancer, and 11 leukemia patients were immunoprecipitated by mouse IgG-Affigel (as a control) or MAb 228-Affigel prior to immunodetection with either rabbit IgG (control) or rabbit anti-CEA antibodies. As presented in the table in Figure 2, 100% (48/48) of the cancer patient samples tested expressed only the 90 kDa glycoform in their serum. The semi-quantitative immunoprecipitation of NCA 90 from patient sera, as determined by intensity of immunoblot staining, was positively correlated with the total NCA 50/90 levels in the serum as measured by ELISA (data not presented). Exclusivity of NCA 90 expression in serum was observed regardless of the total NCA 50/90 serum concentration. Samples with clinically high (> 35 ng/ml) or low (< 35 ng/ml) concentrations of NCA 50/90 expressed only the 90 kDa glycoform.
***Expression of NCA 50/90 In the Serum of Patients with Non-Malignant Diseases*****.** Immunoprecipitation of NCA 50/90 glycoforms from cancer patients serum revealed the presence of only the 90 kDa glycoform in patient sera. To examine the NCA 50/90 species present in the serum of patients with infectious and non-malignant diseases, serum from patients with hepatitis A viral infections, hepatitis B viral infections, cirrhosis, colitis, and pulmonary disease were immunoprecipitated with mouse IgG-Affigel and MAb 228-Affigel. The results, summarized in Figure 2, clearly demonstrate the detection of only the NCA 90 glycoform in the serum of patients with these infectious and non-malignant diseases. CA 228 ELISA values for this patient population ranged from 27.6 to 49.3 ng/ml. Two patient samples (both hepatitis patients) possessed serum NCA 50/90 concentrations which were greater than the upper limit of normal which is 35 ng/ml. In both of these samples, only the 90 kDa glycoform was detected.
***Expression of NCA 50/90 Glycoforms in the Serum of Normal Subjects*****.** Ten serum samples from normal subjects were also subjected the MAb 228-mediated immunoprecipitation and immunoblotting. In all normal serum samples tested, only the NCA 90 glycoform was detected (Figure 2). The CA 228 ELISA values for these samples range from 13.4 to 49.4 ng.ml. Three patients possessed serum NCA 50/90 concentrations which were greater than 35 ng/ml; all three possessed only the 90 kDa glycoform in their serum. These results clearly demonstrate, that like the malignant and non-malignant disease patients, normal subjects possess only the 90 kDa glycoform of NCA 50/90 in the circulation despite the presence of both the 50 kDa and 90 kDa glycoforms in normal lung, colon, and liver tissue.
***Quantitation of NCA 90 in the Sera of Normal Individuals and Patients with Colon, Lung, and Breast Cancer*****. A** method was developed for the quantitation of NCA 90 in normal and cancer patient sera. The method involves isolation of NCA 90 from serum by immunoprecipitation using the MAb 228-Affigel conjugate, electroblotting the precipitate onto PVDF membranes, detecting the transferred precipitate with a polyclonal rabbit anti-CEA antibody preparation, and densitometric scanning of the observed NCA 90 band. Quantitation of immunoprecipitated NCA 90 is performed by comparison of the immunoprecipitated sample's NCA 90 peak area (obtained from the membrane scan) to a standard curve derived from serial dilutions of lung tissue homogenate of known NCA 90 concentration. A representative standard curve of NCA 90 concentration versus NCA 90 peak area is presented in Figure 3.

Using the NCA 90 immunoprecipitation-Western blotting technique, the NCA 90 concentration in the sera of 10 normal and 9 cancer patients (4 colon cancer, 2 lung cancer, and 3 breast cancer patients) was determined and compared to the CA 228 ELISA values determined for these samples. Quantitation of serum NCA 90 by immunoprecipitation-Western blotting yielded mean values of 0.7 ± 0.4 ng/ml (± 1 SD) and 3.5 ± 2.4 ng/ml for normal and cancer patient populations respectively. CA 228 ELISA results for these two patient populations resulted in mean NCA 50/90 levels of 29.3 ± 12.3 ng/ml and 329.4 ± 264.9 ng/ml. The results are presented in Figure 4. Although immunoprecipitation and immunoelectroblotting detects only a fraction of the total NCA 90 present in patient serum, the results depicted in Figure 4 demonstrate that the specific quantitation of serum NCA 90 appears to be a better discriminator of the normal versus the malignant population than does a measurement of total serum NCA 50/90.

A key finding of our studies is detection of only the 90 kDa glycoform of NCA 50/90 in the the circulation. MAb-228 Affigel immunoprecipitation of malignant, non-malignant, infectious disease, and normal serum, resulted in the exclusive detection of NCA 90 in every sample tested. Lung, liver, and colon cancer patients with elevated total serum levels of NCA 50/90, possessed only NCA 90 in the serum. Hepatitis and colitis patients, whose disease is associated with a great deal of tissue damage, also possessed only NCA 90 in the circulation. Several interesting biological hypotheses exist which might explain the exclusive detection of low amounts NCA 90 in normal serum and high amounts of NCA 90 in the serum of a patient with cancer. NCA 50/90-producing cells, either host (granulocytes and/or normal tissue) or tumor cells, synthesize, express on the surface of vesicles, and secrete both glycoforms of NCA 50/90, but only the highly glycosylated NCA 50 form is detected in the circulation due to proteolytic cleavage of the moderately glycosylated NCA 90 form. Preliminary evidence from our laboratory suggests that this is not the case. Spiking of commercially-purified placental NCA 50/90 into normal human serum and subsequent MAb 228-Affigel immunoprecipitation recovered both NCA 50/90 glycoforms (data not presented). Alternatively, cells could synthesize both glycoforms, but only the 90 kDa form is expressed on the surface of the plasma membrane. This hypothesis implies that glycosylation of the NCA 50/90 gene product may direct the cellular localization of the polypeptide; possibly by insuring the proper orientation of the glycoprotein in the lipid bilayer of the cell or membrane vesicle. As a third hypothesis, both glycoforms of NCA 50/90 could be synthesized and expressed, but only the 90 kDa form is secreted (or released) extracellularly. This hypothesis implies that glycoslyation functions as a critical accessory to secretion/release possibly through facilitation of the action of an extracellular glycosyl phosphatidly lipase.

The present invention has been particularly described and exemplified above. Clearly, many other variations and modifications of the invention can be made without departing from the spirit and scope hereof.

### BIBLIOGRAPHY

1. Thomas, P., Toth, C.A., Saini, K.S., Jessup, J.M., and Steele, J. The structure, metabolism and function of the carcinoembryonic antigen gene family. Biochim. et Biophys. Acta, *1032*: 177, 1990.
2. Thompson, J.A., Grunert, F., and Zimmerman, W. Carcinoembryonic antigen gene family: molecular biology and clinical perspectives. J. Clin. Lab. Anal., *5*: 344, 1991.
3. Stanners, C.P., Rojas, M., Zhou, H., Fuks, A., and Beauchemin, N. The CEA family: a system in transitional evolution? Int. J. Biological Markers, *7*: 137, 1992.
4. Nagel, G., and Grunert, F. From genes to proteins: the nonspecific cross-reacting antigens. Tumor Biol., *16*: 17, 1995
5. Khan, W., Frangsmyr, L., Teglund, S., Isrealsson, A., Bremer, K., and Hammarstrom, S. Identification of three new genes and estimation of the size of the carcinoembryonic gene family. Genomics, *14*: 384, 1992.
6. Nagel, G., Grunert, F., Kuijpers, T., Watt, S.M., Thompson, J., and Zimmerman, W. Genomic organization, splice variants and expression of CGM1, a CD 66-related member of the carcinoembryonic antigen gene family. Eur. J. Biochem., *214*: 27, 1993.
7. Kuroki, M., Arakawa, F., Matsuo, Y., Oikawa, S., Misumi, Y., Nakazato, H., and Matsuoka, Y. Molecular cloning of nonspecific crossreacting antigens in human granulocytes. J. Biol. Chem., *266*: 11810, 1991.
8. Khan, W., Teglund, S., Bremer, K., and Hammarstrom, S. The pregnancy-specific glycoprotein family of the immunoglobulin superfamily: identification of new members and estimation of family size. Genomics, *12*: 780, 1992.
9. Barnett, T.R., Pickle, W., and Elting, J.J. Characterization of two new members of the pregnancy-specific β1-glycoprotein family from the myeloid cell line KG-1 and suggestion of two distinct classes of transcription unit. Biochemistry, *29*: 10213, 1990.
10. Olsen, A., Teglund, S., Nelson, D., Gordon, L., Copeland, A., Georgescu, A., Carrano, A., and Hammarstrom, S. Gene organization of the pregnancy-specific glycoprotein region on human chromosome 19: assembly and analysis of a 700-kb cosmid contig spanning the region. Genomics, *23*: 269, 1994.
11. Hasegawa, T., Isobe, K., Tsuchiya, Y., Oikawa, S., Nakazato, H., Nakashima, I., and Shimokata, K. Nonspecific crossreacting antigen (NCA) is a major member of the carcinoembryonic antigen (CEA)-related gene family expressed in lung cancer. Br. J. Cancer, *67*: 58, 1993.
12. Kim, J., Kaye, F.J., Henslee, J.G., Shively, J.E., Park, J-G., Lai, S-L., Linnoila, R.I., Mulshine, J.L., and Gadzar, A.F. Expression of carcinoembryonic antigen in lung and gastrointestinal cancers. Int. J. Cancer, *52*: 718, 1992.
13. Chi, K., Jessup. J. M., and Frazier, M. L. Predomonant expression of mRNA coding for nonspecific cross-reacting antigen in colorectal carcinomas. Tumor Biol., *12*: 298, 1991.
14. Sato, C., Miyaki, M., Oikawa, S., Nakazato, H., and Kosaki, G. Differential expression of carcinoembryonic antigen and nonspecific crossreacting antigen genes in human colon adenocarcinomas and normal colon mucosa. Jpn. J. Cancer Res., *79*: 433, 1988.
15. Cournoyer, D., Beauchemin, N., Boucher, D., Benchimol, S., Fuks, A., and Stanners, C. P. Transcription of genes of the carcinoembryonic antigen family in malignant and nonmalignant human tissues. Cancer Res., *48*: 3153, 1988.
16. Keep, J. C., Benson, A. B., Sullivan, M. D., Carney, W. P., Rosen, S. T., and Radosevich, J. A. Immunophenotypic analysis of colorectal carcinomas with monoclonal antibodies 47D10 and anti-carcinoembryonic antigen. Tumor Biol., *10*: 153, 1989.
17. Nap, M., Keuning, H., Burtin, P., Costerhuis, J. W., and Fleuren, G. CEA and NCA in benign and malignant breast tumors. American J. of Clin. Pathol., *82*: 536, 1984.
18. Radosevich, J. A., Robinson, P. G., Carney, W. P., Warren, W., Rosen, S. T., and Gould, V. E. Immunohistochemical analysis of pulmonary and pleural neoplasms using a monoclonal antibody (47D10) which reacts with a nonspecific cross-reacting antigen. Tumor Biol., *10*: 281, 1989.
19. Kuroki, M., Ichiki, S., Kuroki, M., and Matsuoka, Y. Coproduction of carcinoembryonic antigen and nonspecific cross-reacting antigen by a continuous cell line from a human pancreatic tumor. J. Natl, Cancer Inst., *69*: 401, 1982.
20. Barnett, T., Goebel, S., Nothdurft, M.A., and Elting, J. Carcinoembryonic antigen family: characterization of cDNAs coding for NCA and CEA and suggestion of nonrandom sequence variation in their conserved loop domains. Genomics, *3*: 59, 1988.
21. Barnett, T.R., Kretschmer, A., Austen, D.A., Goebel, S.J., Hart, J.T., Elting, J.J., and Kamarck, M.E. Carcinoembryonic antigens: alternative splicing accounts for the multiple mRNAs that code for novel members of the carcinoembryonic antigen family. J. Cell Biol., 108: 267, 1989.
22. Allard, W.J., Neaman, I.E., Elting, J.J, Barnett, T.R., Yosimura, H., Fritsche, H.A., and Yeung, K.K. Nonspecific cross-reacting antigen 50/90 is elevated in patients with breast, lung, and colon cancer. Cancer Res., *54*: 1227, 1994.
23. Allard, W.J., Neaman, I.E., Very, D.L., Jr., Murray, B.L., and Yeung, K.K. Expression of CEA family members in granulocytes and lymphocytes and use as serum biomarkers in chronic myeloid and chronic lymphocytic leukemias. Manuscript in preparation. 1995.
24. Robbins, P.F., Eggensperger, D., Qi, C-F., and Schlom, J. Definition of the expression of the human carcinoembryonic antigen and non-specific cross-reacting antigen in human breast and lung carcinomas. Int. J. Cancer, *53*: 892, 1993.
25. Baranov, V., Yeung, M.M., and Hammerstrom, B. Expression of carcinoembryonic antigen and nonspecific crossreacting 50 kDa antigen in human normal and cancerous colon mucosa: comparative ultrastructural study with monoclonal antibodies. Cancer Res., *54*: 3305, 1994.
26. Allard, W.J., Vicario, P.P., Saperstein, R., Slater, E.E., and Strout, H.V. The function but not the expression of rat liver inhibitory guanine nucleotide binding protein is altered in streptozotocin-induced diabetes. Endocrinology, *129*: 169, 1991.
27. Allard, W.J., and Lienhard, G.E. Monoclonal antibodies to the glucose transporter from human erythrocytes. Identification of the transporter as a Mᵣ = 55,000 protein. J. Biol. Chem., *260*: 8668, 1985.
28. Kuroki, M., Matsuo, Y., Kinugara, T., and Matsuoka, Y. Augmented expression and release of nonspecific cross-reacting antigens (NCAs), members of the CEA gene family, by human neutrophils during cell activation. J. Leuk. Biol., *52*: 551, 1992.
29. Mayne, K.M., Pulford, K., Jones, M., Micklem, K., Nagel, G., Van der Schoot, C.E., and Mason, D.Y. Antibody By 114 is selective for the 90 kD PI-linked component of the CD66 antigen: a new reagent for the study of paroxysmal nocturnal haemoglobinuria. Brit. J. Haematol., *83*: 30, 1993.
30. Ducher, T.P., and Skubitz, K.M. Subcellular localization of CD66, CD67, and NCA in human neutrophils. J. Leuk. Biol., *52*: 11, 1992.

## Claims

1. A method for measuring the human protein expressed by the NCA 50/90 gene which is secreted into the bloodstream of a patient, comprising measuring NCA 90 in a blood sample obtained from the patient, preferably by performing a sandwich immunoassay in which at least one of the antibody reagents is specific for NCA 90 with no substantial reactivity for NCA 50, CEA, NCA 95, or BGP, and preferably is a monoclonal antibody reagent.

2. In a method for aiding in the diagnosis of cancer in a symptomatic patient, comprising the steps of determining the amount of NCA 50/90 in a blood sample obtained from said patient and comparing said measured amount of NCA 50/90 to the mean blood level of NCA 50/90 in the normal population, whereby the presence of a significantly increased level of NCA 50/90 in the patient's blood is an indication of cancer in the patient,
the improvement which comprises measuring NCA 90 specifically in the patient's blood sample preferably by performing a sandwich immunoassay in which at least one of the antibody reagents is specific for NCA 90 with no substantial reactivity for NCA 50, CEA, NCA 95, or BGP, and preferably is a monoclonal antibody reagent.

3. In a method for monitoring the course of disease in a patient diagnosed with cancer, comprising the performance of a series of specific immunoassays over time to determine changes in the level of NCA 50/90 in blood samples obtained from such patient, whereby changes in the NCA 50/90 blood level correlate with changes in disease status,
the improvement which comprises measuring NCA 90 specifically in the patient's blood sample, preferably by performing a sandwich immunoassay in which at least one of the antibody reagents is specific for NCA 90 with no substantial reactivity for NCA 50, CEA, NCA 95, or BGP, and preferably is a monoclonal antibody reagent.

4. The method of claim 3 wherein the cancer is breast cancer, colon cancer, lung cancer, or leukemic cancer.

5. The method of claim 3 wherein the cancer is breast cancer.

6. In a method for monitoring the course of disease in a patient who has been treated for cancer, comprising the performance of a series of specific immunoassays over time to determine changes in the level of NCA 50/90 in blood samples obtained from such patient, whereby increases in blood NCA 50/90 levels indicate recurrence of disease,
the improvement which comprises measuring NCA 90 specifically in the patient's blood sample, preferably by performing a sandwich immunoassay in which at least one of the antibody reagents is specific for NCA 90 with no substantial reactivity for NCA 50, CEA, NCA 95, or BGP, and preferably is a monoclonal antibody reagent.

7. The method of claim 6 wherein the cancer is breast cancer, colon cancer, lung cancer, or leukemic cancer.

8. The method of claim 6 wherein the cancer is breast cancer.
